# EUROPEAN PATENT APPLICATION

(11) **EP 3 047 819 A1**
(43) Date of publication of application: **27.07.2016**
(21) Application number: 13893562.2
(22) Date of filing: 16.09.2013
(51) Int. Cl.: A61F 2/95, A61F 2/82, A61M 25/01

(54) **SELF-EXPANDING STENT TRANSFER DEVICE**

(71) Applicant: Industry-Academic Cooperation Foundation, Yonsei University, Seoul 120-752 (KR)
(72) Inventor: KIM, Dong Joon, Seoul 137-859 (KR); SHIN, Kyoung Min, Seoul 120-788 (KR); KIM, E. Jason, Goyang-si Gyeonggi-do 410-760 (KR); LEE, Rich, Ganghwa-gun Incheon 417-823 (KR); JUNG, Martin, Gimpo-si Gyeonggi-do 415-719 (KR); KIM, Dong Ik, Seoul 122-737 (KR); KIM, Byung Moon, Seoul 135-926 (KR)
(74) Representative: Gevers & Orès
(86) International application number: PCT/KR2013/008367
(87) International publication number: WO 2015/037766

(57) **Abstract**

Disclosed herein is a delivery apparatus for delivering a shelf-expanding stent to a target position along the inside of a microcatheter, the delivery apparatus including an outer tube configured to communicate with the microcatheter, a shaft part configured to pass through the microcatheter while moving forwards and backwards in the outer tube and including a distal marker and a proximal marker to specify a position of the stent at the target position, and an elastic coating part formed on the outer surface of the shaft part between the distal marker and the proximal marker.

## Description

### [Technical Field]

The present invention relates to a stent delivery apparatus, and more particularly to a delivery apparatus for a self-expanding stent which may be easily deployed and recaptured.

### [Background Art]

In general, the term "vascular disease" commonly refers to diseases caused by blood vessel abnormalities, and abrupt cerebral vascular disorders cause local neurological disorders, such as decreased consciousness, hemiplegia, or language disorder, and, in severe cases, may cause death.

As cerebral vascular diseases, there are an ischemic disease, such as cerebral infarction, which occurs due to blood circulatory disturbances when thrombus are attached to a narrowed region of a cerebral artery and the artery is clogged, and a hemorrhagic disease, such as subarachnoid hemorrhage, which occurs due to cerebral aneurysm in that a part of a blood vessel is swollen.

In order to treat such cerebral vascular diseases, balloon angioplasty, in which a balloon is inserted into a blood vessel using a balloon catheter, fixed to a lesion part of the blood vessel and then expanded so as to enlarge the lesion part, is used. However, such a method may cause problems, including pain experienced by a patient due to the invasive nature of the operation and relapse of the disease. Further, if a cerebral vascular disease is treated through surgery, there is a high risk due to the psychological characteristics of the brain when compared to other organs, and thus, instead of direct surgery, a minimally invasive surgery treatment method is required.

In order to overcome such problems, there is a method using a stent, in which a stent formed of a metal net is inserted into a stenotic blood vessel so as to expand the stenotic blood vessel to its original size or to prevent the further entry of thrombus or flowing blood into the cerebral aneurysm, whereby the blood vessel is treated and normal blood flow is maintained.

Such a stent is a metal net-shaped implant which is inserted into a clogged part through an interventional treatment method rather than surgery, if there is a disorder of the flow of blood or blood fluids, and is used to normalize the flow or to prevent the additional introduction of thrombus or flowing blood into a cerebral aneurysm.

Stent intervention, i.e., a minimally invasive surgery treatment method using a stent, is a treatment method in which, after a micro tube (catheter) or a thin wire (guide wire) is inserted into a blood vessel under X-ray fluoroscopy and approaches a vascular lesion part, a passage of the vascular lesion part is secured using a metal coil so as to normalize the blood flow, or a neck part, i.e., an inlet of a cerebral aneurysm, is closed, and thus the introduction of thrombus and flowing blood into the cerebral aneurysm is prevented so as to normalize blood flow.

Further, as the circumstances require, in order to prevent such a hemorrhagic disease, a treatment method, in which a stent is deployed at a position where a blood vessel may swell so as to prevent the blood vessel from expanding any further, may be used.

U.S. Patent No. 6,019,778 discloses a delivery apparatus for a self-expanding stent, including a shaft as an inner tube and a sheath as an outer tube. A distal tip is mounted at the distal end of the shaft and the stent is provided adjacent to the distal tip. The stent is interposed between the distal tip and a proximal stop and, after the stent and the sheath are delivered to a target position, the stent is deployed at the target position while the sheath is moved.

The proximal stop of the shaft supports the rear end of the stent up to the target position, but, in some cases, the rear end of the stent may be damaged by the stop and the rear end of the stent may not be spread after deployment of the stent.

Further, the stent should be precisely located at the target position, but, even if it is confirmed that the stent is not located at a precise position during a process for moving the sheath, recapture of the stent, i.e., moving of the stent to the inside of the sheath, may not be enabled.

### [Disclosure]

### [Technical Problem]

Therefore, the present invention has been made in view of the above problems, and it is an object of the present invention to provide a stent delivery apparatus which may deliver a self-expanding stent to a target position without damaging the stent.

It is another object of the present invention to provide a stent delivery apparatus which may recapture a self-expanding stent into a sheath loader during a process of deploying the stent and have a wide stent recapturing range.

### [Technical Solution]

In accordance with an aspect of the present invention, the above and other objects can be accomplished by the provision of a delivery apparatus for a self-expanding stent to deliver the self-expanding stent to a target position along the inside of a microcatheter, the delivery apparatus including an outer tube configured to communicate with the microcatheter, a shaft part configured to pass through the microcatheter while moving forwards and backwards in the outer tube, and including a distal marker and a proximal marker to specify a position of the stent at the target position, and an elastic coating part formed on the outer surface of the shaft part between the distal marker and the proximal marker.

The elastic coating part may be in surface contact with the inner surface of the stent within the outer tube and the microcatheter and, even if the stent is exposed to the outside of the microcatheter under the condition that surface contact between the elastic coating part and the stent is maintained, recapture of the stent may be enabled.

The stent within the microcatheter is not supported by a proximal stop but may be mainly in surface contact with the elastic coating part so as to be supported. Therefore, damage to the end of the stent due to the proximal marker or the proximal stop may be prevented, and the stent may be prevented from being pushed between the distal marker and the proximal marker and be moved from an originally fixed position to a target position, i.e., a lesion part.

In a conventional delivery apparatus, if the position of a stent differs from an originally desired position in the process of deploying the stent, it may be difficult to recapture the stent into a microcatheter. However, in the delivery apparatus of the present invention, the elastic coating part may maintain close adhesion with the inner surface of the stent and, thus, the partially exposed stent may be recaptured into the microcatheter simultaneously with restoring of the microcatheter to its original position. When the stent is recaptured, the position of the microcatheter may be accurately adjusted so that the microcatheter is located at the target position by precisely moving the microcatheter and then the microcatheter may accurately deploy the stent at the adjusted position.

The elastic coating part may be formed of an elastic material, such as silicon, urethane and rubber. In order to facilitate recapture of the stent, notched portions having directionality may be formed on the surface of the elastic coating part. The notched portions may be formed to have a straight or curved shape according to required conditions and design specifications and be formed in the entire section or a portion of the section of the elastic coating part. As circumstances require, the notched portions may be formed to have specific directionality. Here, directionality of the notch portions may include a state in which the notch portions are disposed so as to be inclined at a designated angle.

The shaft part may be provided as a guide wire or a separate hollow flexible tube, and the elastic coating part may be formed on the entirety or some portions of a section between the distal marker and the proximal marker and be continuously or discontinuously formed between the distal marker and the proximal marker.

For convenience of working, the delivery apparatus may be provided integrally with a Y-type connector. For this purpose, the delivery apparatus may further include a Y-type connector sealingly connected to the outer tube, and a holder formed at a distal end of the outer tube and fixed to a luer lock of the microcatheter, and a proximal end of the outer tube may be located in a crossing area of the Y-type connector so that, if a saline solution is injected through a syringe, the saline solution may be introduced directly into the microcatheter through the outer tube.

### [Advantageous Effects]

A stent delivery apparatus in accordance with the present invention may deliver a self-expanding stent to a target position without damaging the self-expanding stent. That is, since the stent is in surface contact with an elastic coating part and is supported by the elastic coating part within a microcatheter, damage to the end of the stent by a proximal marker or a proximal stop may be prevented, and the stent may be prevented from being pushed between a distal marker and the proximal marker and be moved from an originally fixed position to a target position, i.e., a lesion part.

Further, the stent delivery apparatus in accordance with the present invention may recapture the self-expanding stent into a sheath loader in the process of deploying the self-expanding stent and have a wide stent recapturing range. Concretely, if the position of the stent differs from an originally desired position in the process of deploying the stent, the stent may be recaptured into the microcatheter. When the stent is recaptured, the position of the microcatheter may be accurately adjusted so that the microcatheter is located at a target position by precisely moving the microcatheter and then the microcatheter may accurately deploy the stent at the adjusted position.

### [Description of Drawings]

The above and other objects, features and other advantages of the present invention will be more clearly understood from the following detailed description taken in conjunction with the accompanying drawings, in which:
FIG. 1 is a perspective view of a delivery apparatus for a self-expanding stent in accordance with one embodiment of the present invention;
FIG. 2 is an exploded perspective view of the delivery apparatus of FIG. 1;
FIG. 3 is a cross-sectional view of the delivery apparatus of FIG. 1;
FIG. 4 is a view illustrating a distal end of a microcatheter at a target position along the delivery apparatus of FIG. 1;
FIG. 5 is a view illustrating a process for deploying a stent in the delivery apparatus of FIG. 1;
FIG. 6 is a view illustrating a process for recapturing a partially exposed stent in the delivery apparatus of FIG. 1;
FIG. 7 is a perspective view illustrating a delivery apparatus for a self-expanding stent in accordance with a further embodiment of the present invention;
FIG. 8 is a view illustrating an elastic coating part of a delivery apparatus in accordance with another embodiment of the present invention;
FIG. 9 is a view illustrating a modification of the elastic coating part of the delivery apparatus in accordance with another embodiment of the present invention;
FIG. 10 is a view illustrating elastic coating parts of a delivery apparatus in accordance with still another embodiment of the present invention; and
FIG. 11 is a view illustrating a delivery apparatus in accordance with yet another embodiment of the present invention.

### [Best Mode]

Hereinafter, preferred embodiments of the present invention will be described in detail with reference to accompanying drawings. In the following description of the present invention, the same or similar elements are denoted by the same reference numerals even though they are depicted in different drawings. In addition, a detailed description of known functions and configurations incorporated herein will be omitted when it may make the subject matter of the present invention rather unclear.

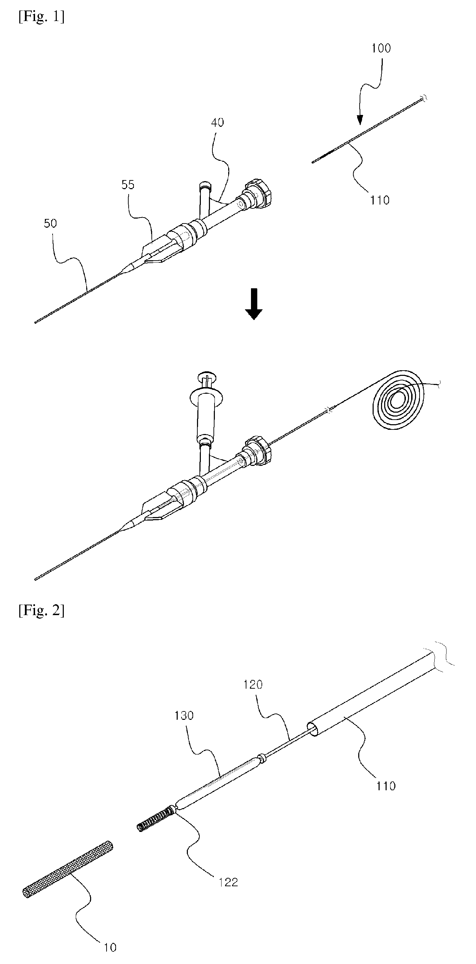

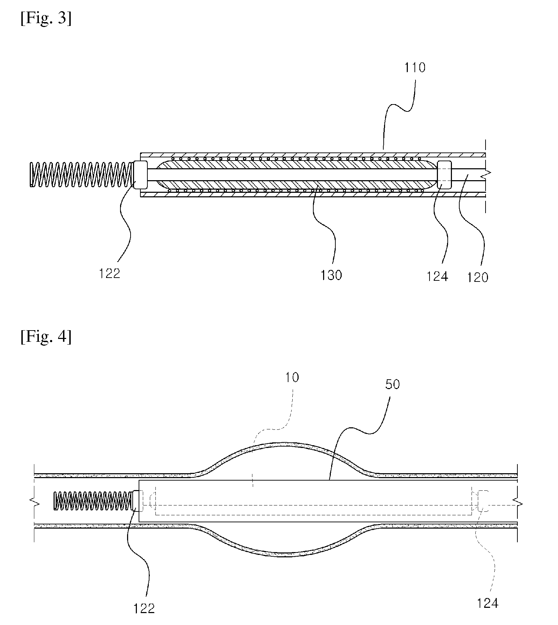

FIG. 1 is a perspective view of a delivery apparatus for a self-expanding stent in accordance with one embodiment of the present invention, FIG. 2 is an exploded perspective view of the delivery apparatus of FIG. 1, and FIG. 3 is a cross-sectional view of the delivery apparatus of FIG. 1.

With reference to FIGS. 1 to 3, a delivery apparatus 100 for a self-expanding stent 10 may include an outer tube 110, a shaft part 120 and an elastic coating part 130, and be connected to a microcatheter 50 which is inserted into a human body through the skin. The microcatheter 50 may be already inserted into to a target position, i.e., a lesion part, and, as needed, may be moved together with the shaft part 120.

The outer tube 110 may be formed to have an inner diameter of about 0.60 to 0.75 mm and communicate with the microcatheter 50. The shaft part 120 and the stent 10 may be mounted in advance in the outer tube 110. The stent 10 has an extensible and contractible structure of an almost cylindrical shape by interconnecting or twisting fine wires. Although, as one example, the stent 10 may have a diamond-shaped net structure, the stent 10 may have a zigzag net shape in the same manner as other conventional stents (with reference to U.S. Patent No. 6,019,778) or other shapes. For reference, the outer tube 110 may be provided so as to match the dimensions of the microcatheter 50.

The shaft part 120 together with the stent 10 may be located within the outer tube 110 and be moved to a target position via the microcatheter 50. The shaft part 120 may be formed of a material harmless to human bodies, or be formed of a metal, such as stainless steel, or an artificial material harmless to human bodies, such as polyamide or polyurethane. The shaft part 120 may be formed to have a diameter or thickness of about 0.10 to 0.20 mm.

The shaft part 120 may include a distal marker 122 and a proximal marker 124 so as to specify the position of the stent 10 at the target position. Further, the elastic coating part 130 surrounding the outer surface of the shaft part 120 is provided between the distal marker 122 and the proximal marker 124. The elastic coating part 130 may be formed of an elastic material, such as silicon, urethane or rubber, be in surface contact with the inner surface of the stent 10, and transmit frictional force so that the stent 10 may smoothly move within the microcatheter 50 and the outer tube 110. Differently from other conventional delivery apparatuses in which a proximal stop corresponding to the proximal marker 124 supports the rear end of a stent while the stent is delivered, in the delivery apparatus in accordance with this embodiment, the elastic coating part 130 having a thickness of about 0.20 to 0.25 mm is in surface contact with the inner surface of the stent 10 and thus forms a friction surface, and contacts the entire inner surface of the stent 10 so as to move the stent 10 without damaging portions of the stent 10.

As one example, when the stent 10 is mounted at the shaft part 120 and then the shaft part 120 is inserted into the outer tube 110, the shaft part 120 and the elastic coating part 130 may have a thickness of about 0.58 mm and the stent 10 may be formed of fine wires having a diameter of about 0.03 mm and thus have a thickness of 0.06 mm at most. Therefore, the total thickness of the shaft part 120 and the elastic coating part 130 and the stent 10 may be about 0.70 mm and they may be inserted into the outer tube 110 having an inner diameter of about 0.69 mm. Here, when the shaft part 120, the elastic coating part 130 and the stent 10 are mounted within the external tube 110, the total thickness of the shaft part 120 and the elastic coating part 130 and the stent 10 may be equal to the inner diameter of the outer tube 110 or differ from the inner diameter of the outer tube 110 by about 0.01 mm.

A coil-shaped guide part may be provided at the distal marker 122 or adjacent to the distal marker 122. The distal marker 122 and the proximal marker 124 may be formed of a material or have a shape which enables measurement of the position of the stent 10 within a blood vessel of a human body through an X-ray image or other measurement methods.

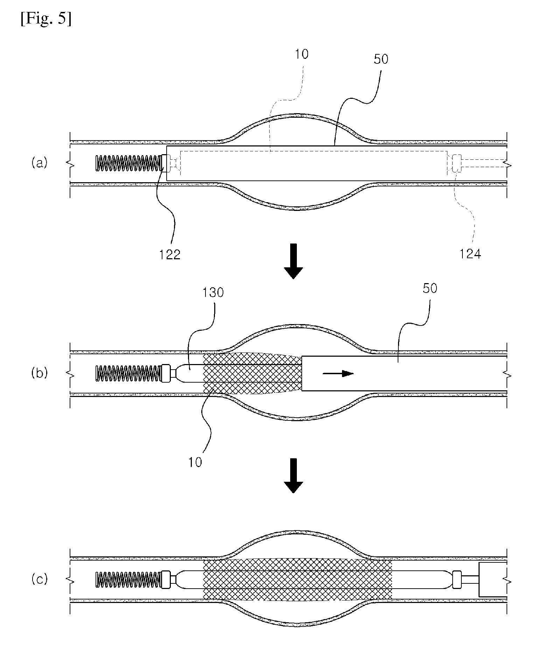

FIG. 4 is a view illustrating the distal end of the microcatheter at a target position along the delivery apparatus of FIG. 1, and FIG. 5 is a view illustrating a process for deploying the stent in the delivery apparatus of FIG. 1.

With reference to FIG. 4, the shaft part 120 and the stent 10 may move along the microcatheter 50. The position of the stent 10 may be specified by the distal marker 122 and the proximal marker 124 and the stent 10 may be deployed at a position in a blood vessel which is swollen or is in danger of swelling.

With reference to FIG. 5, after the stent 10 is located at a target position, the microcatheter 50 may be relatively retracted (a). Since the stent 10 is fixed to the elastic coating part 130 with a relatively large frictional force, only the microcatheter 50 may be retracted, and the stopped shaft part 120 and stent 10 at the fixed position may be exposed from the microcatheter 50 to the outside.

As the stent 10 starting from the end thereof is exposed, the stent 10 itself expands, the expanding stent 10 may be closely attached to the inner wall of the blood vessel and, thus, a space may be formed by the expanding stent 10. The stent 10 expands starting from the exposed front end thereof and, as the microcatheter 50 is retracted, the extent to which the stent 10 is exposed and expanded is gradually increased (b).

When the stent 10 is completely deployed from the microcatheter 50, the stent 10 forms a cylindrical space in the inner wall of the blood vessel and may thus prevent the blood vessel from continuously swelling due to a blood pressure. Of course, if a blood vessel is narrowed by thrombus, the same type of stent or a different type of stent may be used to expand the blood vessel.

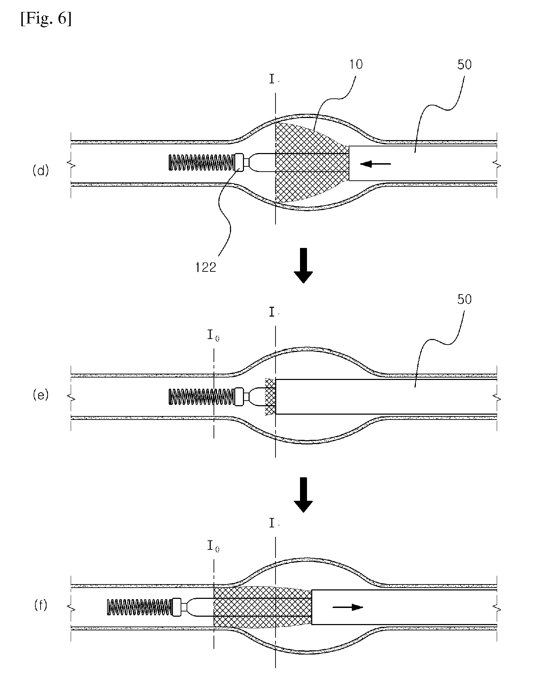

FIG. 6 is a view illustrating a process for recapturing the partially exposed stent in the delivery apparatus of FIG. 1.

With reference to FIG. 6, in the process of exposing the stent 10, the diameter of the stent 10 is increased and the length of the stent 10 is decreased and, thus, it may be difficult to deploy the stent 10 at a desired position. Further, the case in that the stent 10 may be deployed at a position I_{I}, differing from a target position I₀, may frequently occur due to a number of variables generated during the treatment process (d).

In this case, by moving the microcatheter 50 forwards again, the stent 10 may be inserted into the microcatheter 50. Here, a part of the stent 10, which is not deployed from the microcatheter 50, may maintain close adhesion with the elastic coating part 130 and maintain effective frictional force. Therefore, if the microcatheter 50 is moved forwards for recapturing the stent 10, friction between the elastic coating part 130 and the rear end of the stent 10 may be maintained, and the stent 10 may be recaptured into the microcatheter 50 without pushing the stent 10 and without the stent 10 escaping from the microcatheter 50.

For reference, in a conventional stent delivery apparatus not using elastic coating, a partially expanding stent may not be recaptured by a microcatheter and, even if the microcatheter is moved forwards, the stent is not recaptured by the microcatheter and there is a high possibility that the stent will be pushed and thus deployed at an unexpected position.

Referring to the figures again, when the stent 10 is recaptured by the microcatheter 50 so as to be located within the microcatheter 50, the stent 10 may be moved to the target position I₀ using the distal marker and the proximal marker and be deployed at a precise position, as exemplarily shown in FIG. 5.

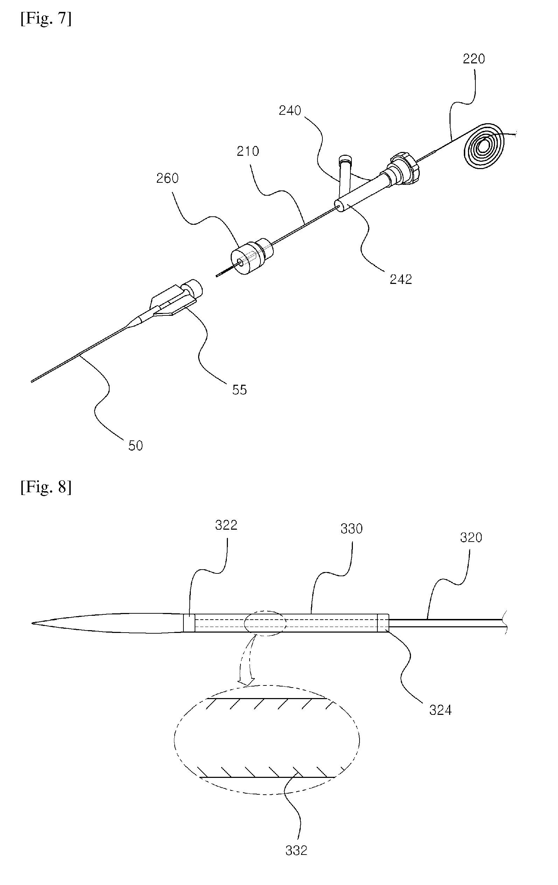

FIG. 7 is a perspective view illustrating a delivery apparatus for a self-expanding stent in accordance with a further embodiment of the present invention.

With reference to FIG. 7, the delivery apparatus includes a Y-type connector 240, an outer tube 210 mounted at the front part of the Y-type connector 240, a holder 260 mounted at a distal end of the outer tube 210 and fixed to a luer lock 55 of a microcatheter 50, a shaft part 220 moving to a lesion part through the microcatheter 50, and an elastic coating part 230 to deliver the stent while maintaining close adhesion with the stent. Here, a proximal end of the outer tube 210 is located in a crossing area 242 of the Y-type connector 240.

In the delivery apparatus, the Y-type connector 240 is provided integrally with the outer tube 210 and the shaft part 220, and preparation for delivery of the stent may be completed together with connection of the Y-type connector 240 without separate fixation of the Y-type connector to the luer lock 55 and separate fixation of the outer tube 210.

Particularly, as exemplarily shown in FIG. 1, prior to delivery of the stent, in order to prevent introduction of air into a blood vessel, a process for injecting a saline solution into the Y-type connector through a branch passage is required. However, in the state shown in FIG. 1, the outer tube 110 disturbs normal introduction of a saline solution and may divert the saline solution but, if the proximal end of the outer tube 210 is located in the crossing area 242 of the Y-type connector 240 provided integrally with the outer tube 210, as exemplarily shown in FIG. 7, there is no possibility of introduction of external air, and injection of a saline solution or some other medicines is facilitated without diversion.

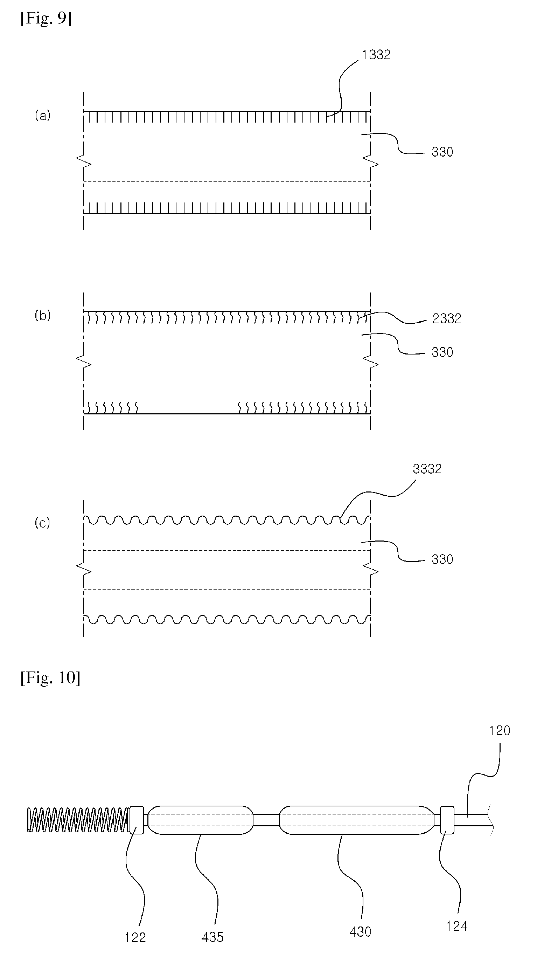

FIG. 8 is a view illustrating an elastic coating part of a delivery apparatus in accordance with another embodiment of the present invention and FIG. 9 is a view illustrating a modification of the elastic coating part of the delivery apparatus in accordance with another embodiment of the present invention.

With reference to FIG. 8, a distal marker 322 and a proximal marker 324 may be provided at ends of a shaft part 320, and a long bullet-shaped guide may be provided adjacent to the distal marker 322. An elastic coating part 330 may be provided between the distal marker 322 and the proximal marker 324, and a plurality of notched portions 332 may be formed on the surface of the elastic coating part 330.

The notched portions 332 may assist stable recapture of a stent and, even if a considerable length of the stent is deployed, the minimum engagement length, which enables recapture of the stent, may be maintained to be shorter so long as contact between the stent and the elastic coating part 330 is maintained.

The notched portions 332 may be provided to have various shapes according to required conditions and design specifications. As one example, with reference to FIG. 8, the notched portions 332 may be formed to have a straight line shape having a fine width, and have directionality so as to face the proximal marker 324, i.e., to face or incline backwards.

As circumstances require, notched portions may be formed to have some other shapes and the width of the notched portions may be suitably changed according to required conditions and design specifications.

As one example, with reference to FIG. 9(a), notched portions 1332 may be formed to have a straight line shape and disposed in the radial direction of the shaft part 320.

As another example, with reference to FIG. 9(b), notched portions 2332 may be formed to have a curved shape, i.e., an approximate S shape. Further, the notched portions 2332 may be formed in the entire section or a portion of the section of the elastic coating part, and the section in which the notched portions 2332 are formed does not limit or restrict the present invention.

As yet another example, with reference to FIG. 9(c), notched portions 3332 may have a width of a designated value or more. If the notched portions 3332 have a width of a designated value or more, the surface of the elastic coating part may be formed to have irregularity in a wavy pattern. For reference, the width of notched portions and the separation distance between the notched portions may be suitably changed according to required conditions and design specifications.

FIG. 10 is a view illustrating elastic coating parts of a delivery apparatus in accordance with still another embodiment of the present invention.

With reference to FIG. 10, an elastic coating part is not formed in the entire section between a distal marker 122 and a proximal marker 124 of a shaft part 120, but elastic coating parts 430 and 435, separated from each other, may be discontinuously formed therebetween.

The discontinuous elastic coating parts 430 and 435 may be provided in various patterns. As one example, two elastic coating parts or three or more elastic coating parts may be provided so as to be separated from each other in the length direction, or the elastic coating parts may be separated from each other in the circumferential direction but not in the length direction.

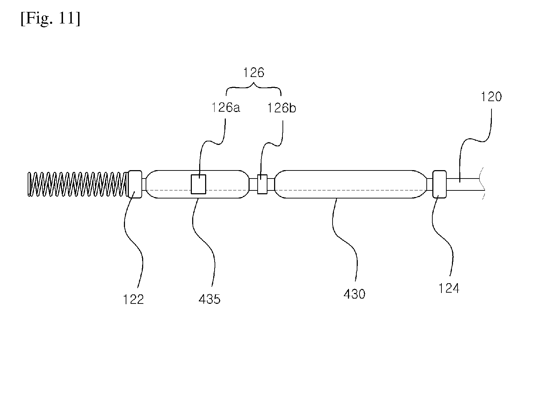

FIG. 11 is a view illustrating a delivery apparatus in accordance with yet another embodiment of the present invention. Hereinafter, some portions in this embodiment, which are substantially the same as or similar to those in the former embodiments, are denoted by the same reference numerals even though they are depicted in different drawings and a detailed description thereof will thus be omitted because it is considered to be unnecessary.

With reference to FIG. 11, the delivery apparatus in accordance with this embodiment may include at least one intermediate marker 126 formed between a distal marker 122 and a proximal marker 124.

The intermediate markers 126 may be formed of a material or have a shape which enables measurement of the position of the stent 10 within a blood vessel of a human body through an X-ray image or other measurement methods in a manner that is the same as or similar to the above-described distal marker 122 and proximal marker 124.

The intermediate markers 126 may be provided at various regions according to required conditions and design specifications, and the number and separation distance of the intermediate markers 126 may be suitably changed according to required conditions.

As one example, a plurality of elastic coating parts 430 and 435 may be discontinuously formed in the length direction between the distal marker 122 and the proximal marker 124, and the intermediate markers 126 may include a first intermediate marker 126a formed on an area, where the elastic coating part 435 is formed, and a second intermediate marker 126b formed between the separated elastic coating parts 430 and 435.

For reference, it may be understood that formation of the first intermediate marker 126a on the area, where the elastic coating part 435 is formed, includes formation of the first intermediate marker 126a on the surface of the elastic coating part 435 or within the elastic coating part 435 in the area, where the elastic coating part 435 is formed.

On the other hand, even if an elastic coating part is continuously formed in the length direction between a distal marker and a proximal marker, a plurality of intermediate markers separated from each other may be formed on the surface of the elastic coating part.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A delivery apparatus for a self-expanding stent to deliver the self-expanding stent to a target position along the inside of a microcatheter, the delivery apparatus comprising:
an outer tube configured to communicate with the microcatheter;
a shaft part configured to pass through the microcatheter while moving forwards and backwards in the outer tube, and including a distal marker and a proximal marker to specify a position of the stent at the target position; and
an elastic coating part formed on the outer surface of the shaft part between the distal marker and the proximal marker,
wherein the elastic coating part is in surface contact with the inner surface of the stent within the outer tube and the microcatheter and, even if the stent is exposed to the outside of the microcatheter under the condition that surface contact between the elastic coating part and the stent is maintained, recapture of the stent is enabled.

2. The delivery apparatus according to claim 1, wherein the elastic coating part is formed of an elastic material including at least one of silicon, urethane and rubber.

3. The delivery apparatus according to claim 1, wherein notched portions are formed on the surface of the elastic coating part.

4. The delivery apparatus according to claim 3, wherein the notched portions are formed to have a straight or curved shape.

5. The delivery apparatus according to claim 3, wherein the notched portions are formed in the entire section or a portion of the section of the elastic coating part.

6. The delivery apparatus according to claim 3, wherein the notched portions have directionality so as to face the proximal marker of the shaft part.

7. The delivery apparatus according to claim 1, wherein the shaft part is a guide wire.

8. The delivery apparatus according to claim 1, wherein the elastic coating part is formed on the entirety or some portions of a section between the distal marker and the proximal marker.

9. The delivery apparatus according to claim 1, wherein the elastic coating part is continuously or discontinuously formed between the distal marker and the proximal marker.

10. The delivery apparatus according to claim 1, further comprising a Y-type connector sealingly connected to the outer tube, and a holder formed at a distal end of the outer tube and fixed to a luer lock of the microcatheter,
wherein a proximal end of the outer tube is located in a crossing area of the Y-type connector.

11. The delivery apparatus according to claim 1, further comprising at least one intermediate marker formed between the distal marker and the proximal marker.

12. The delivery apparatus according to claim 11, wherein the at least one intermediate marker is formed on an area where the elastic coating part is formed.

13. The delivery apparatus according to claim 11, wherein:
a plurality of elastic coating parts is discontinuously formed in the length direction between the distal marker and the proximal marker; and
the at least one intermediate marker is formed between the separated elastic coating parts.
